(19) Europäisches Patentamt
European Patent Office
Office européen des brevets

(11) Publication number: 0 464 975 A2

# (12) EUROPEAN PATENT APPLICATION

(21) Application number: **91250166.5**

(22) Date of filing: **24.06.91**

(51) Int. Cl.5: **C07D 471/04**, A01N 43/90, //(C07D471/04,249:00,221:00)

(30) Priority: **26.06.90 DE 4020629**

(43) Date of publication of application: **08.01.92 Bulletin 92/02**

(84) Designated Contracting States: **AT BE CH DE DK ES FR GB GR IT LI LU NL SE**

(71) Applicant: **SCHERING AKTIENGESELLSCHAFT**
**Müllerstrasse 170/178**
**W-1000 Berlin 65(DE)**

(72) Inventor: **Dorfmeister, Gabriele, Dr.**
**Heiligenseestrasse 70**
**W-1000 Berlin 27(DE)**
Inventor: **Franke, Wilfried, Dr.**
**Spiessergasse 6b**
**W-1000 Berlin 27(DE)**
Inventor: **Ganzer, Michael, Dr.**
**Eichobrndamm 279**
**W-1000 Berlin 26(DE)**

## (54) Process and intermediates for the preparation of bicyclic triazoles.

(57) The invention relates a new process for the preparation of bicyclic triazoles in which of general formula I

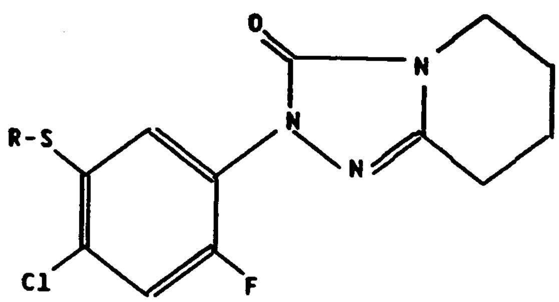

(I)

in which R is $C_1$-$C_6$-alkyl or $C_1$-$C_6$-alkoxycarbonyl, which starts from the known 2-(4-chloro-2-fluorophenyl)-5,6,7,8-tetrahydro-1,2,4-triazolo[4,3-a]pyridin-3(2H)-one of formula II

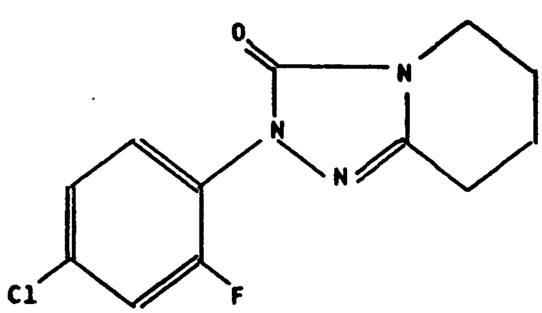

(II)

via the new chlorosulphonyl compound of formula III

EP 0 464 975 A2

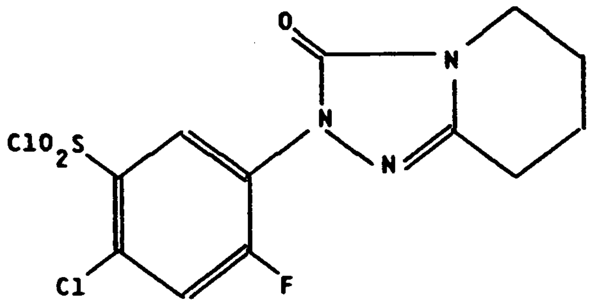

(III)

and via the new mercapto of general formula IV

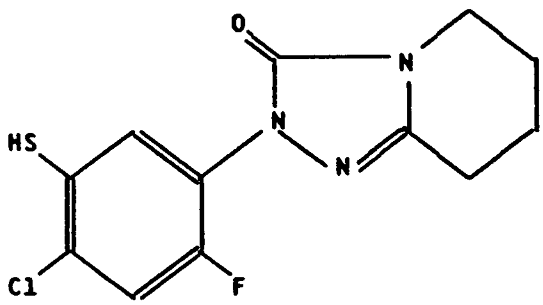

(IV)

This invention relates to a new process and new intermediates for the preparation of bicyclic triazoles of general formula I

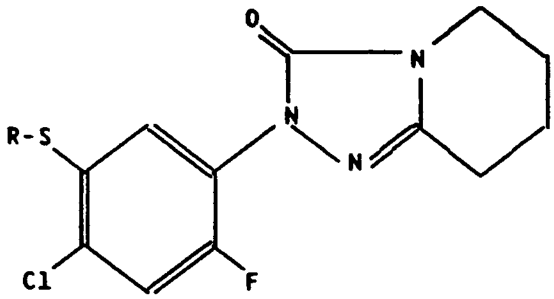

(I)

in which R is $C_1$-$C_6$-alkyl or $C_1$-$C_6$-alkoxycarbonyl.

These compounds have excellent herbicidal activity against a broad spectrum of monocotyledonous and dicotyledonous weeds in agricultural crops. Their preparation and use are described in EP 317 947.

The object of the present invention is to provide a new process which allows preparation of compounds of general formula I without any problems, and under mild reaction conditions.

This object is solved by a process for the preparation of bicyclic triazoles in which

a) a compound of formula II

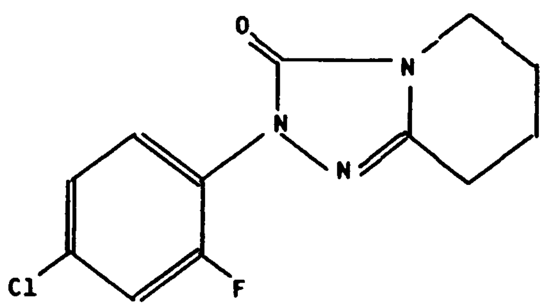

(II)

is reacted with chlorosulphonic acid, in the presence of a mineral acid binding agent,

b) the chlorosulphonyl compound, so formed, of formula III

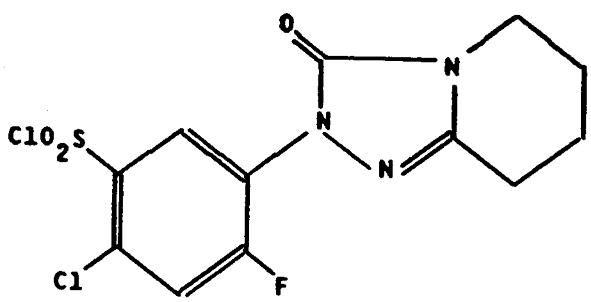

(III)

is reduced with a metal in the presence of an acid, and finally

c) the mercapto compound, so formed, of general formula IV

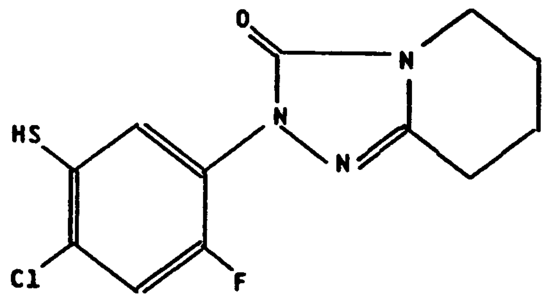

(IV)

is reacted with a compound of general formula V

R-Hal (V)

in which R has the meaning given in general formula I and Hal is halogen, in a solvent in the presence of a base.

The 2-(4-chloro-2-fluorophenyl)-5,6,7,8-tetrahydro-1,2,4-triazolo[4,3-a]pyridin-3(2H)-one of formula II, which is used as the starting material in step a), is known in the literature (DE 28 01 429).

The 2-(4-chloro-5-chlorosulphonyl-2-fluorophenyl)-5,6,7,8-tetrahydro-1,2,4-triazolo[4,3-a]pyridin-3(2H)-one of formula III, which is used as the starting material in step b), is new and is also within the scope of this invention.

The preparation is carried out by reacting the compound of formula II with chlorosulphonic acid, in known manner. The reaction temperature is generally between 0 and 100 °C and the reaction time is usually from 1 to 24 hours.

Surprisingly the reaction of the compound of formula II with chlorosulphonic acid occurs quantitatively. A corresponding disulphone, as described in the literature, (for example in Houben-Weyl, Vol. 9, page 238) is not formed as a by-product.

The 2-(4-chloro-2-fluorophenyl-5-mercapto)-5,6,7,8-tetrahydro-1,2,4-triazolo[4,3-a]pyridin-3(2H)-one of formula IV, which is used as the starting material in step c), is new and is also within the scope of this invention.

The preparation is suitably carried out by reacting the starting material of formula III with a metal, preferably zinc, in an acid, preferably acetic acid. The reaction temperature is generally between 0 and 100 °C and the reaction time is usually from 1 to 24 hours.

Other suitable metals include iron and other suitable acids include sulphuric and hydrochloric acid.

The mercapto compound of formula IV can then be reacted in an similar manner to known processes with a halide of formula V, in a suitable solvent in the presence of a base. The reaction is generally carried out at room temperature and the reaction time is usually from 1 to 24 hours. Suitable solvents include dimethylformamide and tetrahydrofuran and a suitable base is sodium hydride.

The following examples illustrate the invention.

<u>Example 1</u> - Process step a)

2-(4-Chloro-5-chlorosulphonyl-2-fluorophenyl)-5,6,7,8-tetrahydro-1,2,4-triazolo[4,3-a]pyridin-3(2H)-one

50 g 2-(4-Chloro-2-fluorophenyl)-5,6,7,8-tetrahydro-1,2,4-triazolo[4,3-a]pyridin-3(2H)-one was added with ice cooling to 100 ml chlorosulphonic acid and 40 ml oleum added dropwise. The mixture was heated at 90 °C for 5 hours and after cooling added to 800 g ice.The precipitate was filtered, washed with water and dried in vacuo at 40 °C.

Yield: 65.1 g = 98% of theory
Mp: 148 °C (dec.)

<u>Example 2</u> - Process step b)

2-(4-Chloro-2-fluorophenyl-5-mercapto)-5,6,7,8-tetrahydro-1,2,4-triazolo[4,3-a)pyridin-3(2H)-one

65 g 2-(4-Chloro-5-chlorosulphonyl-2-fluorophenyl)-5,6,7,8-tetrahydro-1,2,4-triazolo[4,3-a)pyridin-3(2H)-one was added with ice cooling to 200 ml water and 200 ml concentrated sulphuric acid. 106 g Zinc powder was added portionwise, under vigorous stirring, whilst maintaining the temperature at 30 ° C. After stirring for 12 hours at room temperature, the mixture was filtered and the filtrate extracted with ether. The extract was washed with saturated aqueous sodium chloride, dried over magnesium sulphate and concentrated.

Yield: 24.2 g = 45.4% of theory
Mp: 96 ° C

Example 3.1 - Process step c)

2-(4-Chloro-2-fluoro-5-isopropylphenyl)-5,6,7,8-tetrahydro-1,2,4-triazolo[4,3-a)pyridin-3(2H)-one

0.3 g Sodium hydride (80%) in 50 ml dimethylformamide was treated with 2.8 g 2-(4-chloro-2-fluorophenyl-5-mercapto)-5,6,7,8-tetrahydro-1,2,4-triazolo[4,3-a]pyridin-3(2H)-one and mixture stirred for 30 minutes at room temperature. It was then treated with 100 ml water and 100 ml ether and the phases separated. The aqueous phase was shaken with ether. The combined ether phases were dried over magnesium sulphate and concentrated.

Yield: 3.3 g = 96.5% of theory
Mp: 123 ° C

Example 2.2 - Process step b)

2-(4-Chloro-5-ethoxycarbonyl-2-fluorophenyl)-5,6,7,8-tetrahydro-1,2,4-triazolo[4,3-a]pyridin-3(2H)-one

0.3 g Sodium hydride (80%) in 50 ml tetrahydrofuran was treated with 2.8 g 2-(4-chloro-2-fluorophenyl-5-mercapto)-5,6,7,8-tetrahydro-1,2,4-triazolo[4,3-a]pyridin-3(2H)-one and mixture stirred for 30 minutes at 30 ° C. It was then treated with 100 ml water and 100 ml ether and the phases separated. The aqueous phase was shaken with ether. The combined ether phases were dried over magnesium sulphate and concentrated.

Yield: 3.55 g = 92% of theory
$n_D^{20}$ : 1.5707

## Claims

**1.** A process for the preparation of bicyclic triazoles in which of general formula I

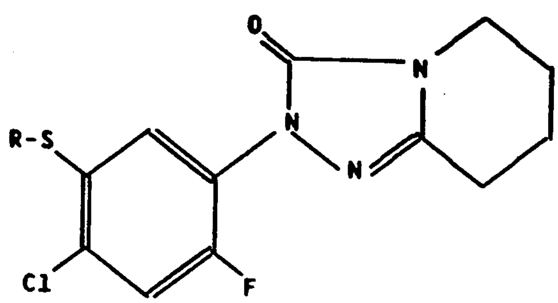

(I)

in which R is $C_1$-$C_6$-alkyl or $C_1$-$C_6$-alkoxycarbonyl, in which
a) a compound of formula II

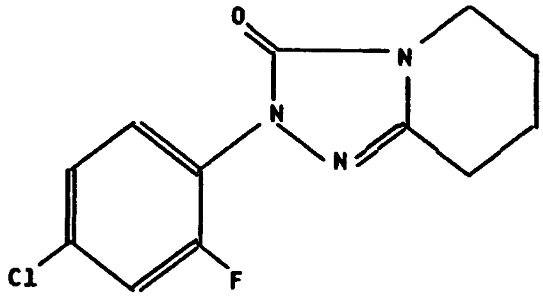

(II)

is reacted with chlorosulphonic acid, in the presence of a mineral acid binding agent,
b) the chlorosulphonyl compound, so formed, of formula III

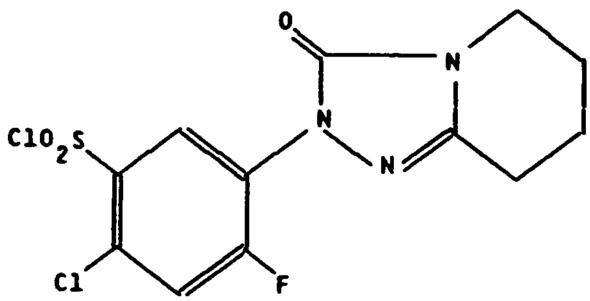

(III)

is reduced with a metal in the presence of an acid, and finally
c) the mercapto compound, so formed, of general formula IV

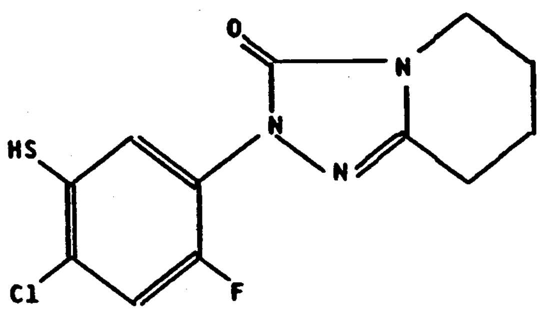

(IV)

is reacted with a compound of general formula V

R-Hal (V)

in which R has the meaning given in general formula I and Hal is halogen, in a solvent in the presence of a base.

**2.** 2-(4-Chloro-5-chlorosulphonyl-2-fluorophenyl)-5,6,7,8-tetrahydro-1,2,4-triazolo[4,3-a]pyridin-3(2H)-one of formula III

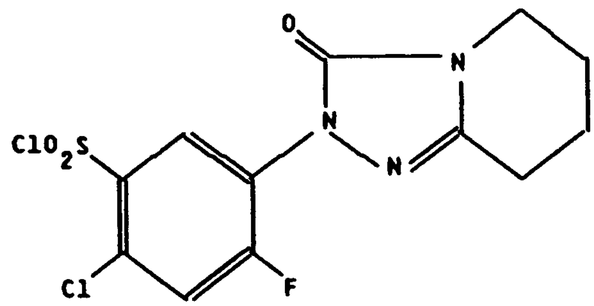

(III)

3. 2-(4-Chloro-2-fluoro-5-mercaptophenyl)-5,6,7,8-tetrahydro-1,2,4-triazolo[4,3-a]pyridin-3(2H)-one of formula IV

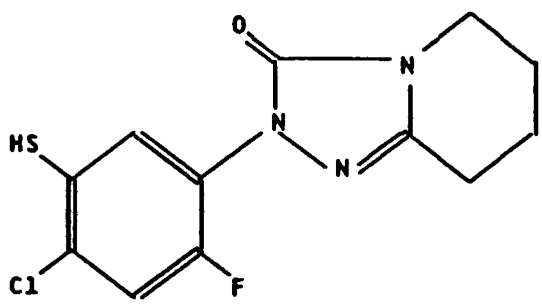

(IV)